# EUROPÄISCHE PATENTSCHRIFT

(11) **EP 2 522 308 B1**
(45) Veröffentlichungstag und Bekanntmachung des Hinweises auf die Patenterteilung: **25.02.2015**
(21) Anmeldenummer: 12164372.0
(22) Anmeldetag: 17.04.2012
(51) Int. Cl.: A61F 2/24

(54) **Mechanische Transkatheter-Herzklappenprothese**
Mechanical transcatheter heart valve prosthesis
Prothèse de valvule mécanique à transcathéter

(30) Priorität: 10.05.2011 US 201161484241 P
(43) Veröffentlichungstag der Anmeldung: 14.11.2012
(73) Patentinhaber: Biotronik AG, 8180 Bülach (CH)
(72) Erfinder: Quint, Bodo, 8154 Oberglatt (CH); Wesselmann, Matthias, 8455 Rüdlingen (CH); Schwitzer, Alwin, 8180 Bülach (CH); Bachmann, Patrice, 8408 Winterthur (CH); Pfenninger-Ganz, Susanne, 8607 Aathal (CH); Lang, Hans, 8107 Buchs (CH); Fargahi, Amir, 8180 Bülach (CH)
(74) Vertreter: Lindner-Vogt, Karin L.

(56) Entgegenhaltungen:
- WO-A1-02/24119
- WO-A1-2009/038761
- US-A1- 2003 078 652
- US-A1- 2006 190 074

## Beschreibung

Die Erfindung betrifft eine mechanische Transkatheter-Herzklappenprothese.

Die Herzscheidewand trennt das menschliche Herz in zwei Hälften und zwar in eine rechte Herzkammer und einen rechten Vorhof sowie in eine linke Herzkammer und einen linken Vorhof. Zwischen den Herzkammer und Vorhöfen befinden sich vier Herzklappen. Das sauerstoffarme, aber kohlendioxidreiche Blut fließt zunächst durch die Trikuspidalklappe in den rechten Vorhof und von dort aus in die rechte Herzkammer. Die Trikuspidalklappe ist eine dreizipflige Klappe und wird auch als Segelklappe bezeichnet. Von der rechten Kammer gelangt das Blut durch die Pulmonalklappe in die beiden Lungenhälften, wo das Blut wieder mit Sauerstoff angereichert wird. Die Pulmonalklappe ist eine sogenannte Taschenklappe. Das mit Sauerstoff angereicherte Blut gelangt nun aus der Lunge in den linken Vorhof und wird durch die Mitralklappe, die die Form einer zweizipfligen Segelklappe besitzt, in die linke Kammer gepumpt. Schließlich gelangt das Blut von der linken Herzkammer aus durch die Aortenklappe in den großen Blutkreislauf. Die Aortenklappe ist wie die Pulmonalklappe eine Taschenklappe.

Bestehen bei einem Patienten Herzklappenfehler, so ist davon auszugehen, dass sich im Laufe der Zeit die Funktion dieser Herzklappen ständig verschlechtern kann. Der Ersatz von nicht mehr funktionsfähigen Herzklappen durch Herzklappenprothesen ist nach der koronaren Bypassoperation mittlerweile die am häufigsten durchgeführte Operation am menschlichen Herzen. Ein idealer Herzklappenersatz sollte dabei eine unbegrenzte Haltbarkeit besitzen, ungehinderte Blutflussbedingungen im Gefäß ermöglichen, keine herzklappenbedingte Komplikationen wie erhöhte Thrombogenität oder Endokarditis-Anfälligkeit bedingen, keine prothesenimmanente Risiken wie klappenbedingte Defekte aufweisen, eine einfache Implantation ermöglichen und geräuscharm sein.

Es werden zwei verschiedene Typen von Herzklappenprothesen eingesetzt, nämlich mechanische und biologische Herzklappenprothesen.

Als biologische Herzklappen werden Prothesen bezeichnet, die aus biologischem Material, insbesondere aus den Aortenklappenflügeln von Schweinen oder dem Herzbeutel von Rindern zusammengesetzt werden. Das Gewebe wird zur Fixierung in der Regel chemisch behandelt und auf einem expandierbaren Stützgerüst zur späteren Fixierung im Herzen befestigt.

Biologische Herzklappenprothesen im eingepflanzten Zustand sind allerdings nur begrenzt haltbar, weil die Verkalkung die Klappen zunehmend beeinflusst. Es ist von einer mittleren Lebensdauer von etwa 10 bis 12 Jahren auszugehen. Besonders ausgeprägt ist die schnelle Verkalkung der biologischen Herzklappen jedoch bei jüngeren Patienten. Daher wird die Implantation von biologischen Herzklappen erst im fortgeschrittenen Alter vorgenommen, um ein zweites Auswechseln der Klappe nicht mehr vornehmen zu müssen. Bei der chemischen Behandlung natürlichen Gewebes für biologische Herzklappen sind ferner erhebliche Anstrengungen notwendig, um die mechanischen Eigenschaften zu erhalten. Die BSE Problematik hat zudem gezeigt, dass der Einsatz von Biomaterialien auch Risiken birgt.

Mechanische Herzklappen haben gegenüber den biologischen Herzklappen den Vorteil der längeren Haltbarkeit. Ein mechanischer Herzklappenersatz bedarf jedoch einer lebenslangen gerinnungshemmenden Therapie.

Mittlerweile sind künstliche Herzklappen entwickelt worden, die eine im Labor nachgewiesene Lebensdauer von 150 Jahren haben. Die hämodynamischen Verhältnisse sind annähernd vergleichbar mit denen natürlicher Herzklappen. Die klappenbedingten Komplikationen wie Gerinnselbildung sind nahezu verschwunden, prothesenbedingte Komplikationen aufgrund einer hervorragenden Weiterentwicklung praktisch nicht mehr aufgetreten. Die mechanischen Herzklappen sind relativ einfach einzupflanzen und das Klappengeräusch der mechanischen Herzklappe wird gut vom Patienten toleriert, da diese - zumindest für die Umwelt - relativ geräuscharm sind.

Viele der derzeit zugelassenen künstlichen Herzklappen können jedoch ausschließlich durch Operation am offenen Herzen implantiert werden, was den Einsatz dieser Prothesen deutlich limitiert, da bei etwas mehr als 1/3 aller Patienten ein hohes Operationsrisiko besteht oder eine solche Operation nicht mehr möglich ist. Aus diesem Grunde sind mittlerweile minimal-invasive Techniken und Transkatheter-Herzklappenprothesen entwickelt wurden, bei denen die neue Herzklappe mit Hilfe eines Kathetersystems an den Implantationsort verbracht und verankert wird (zum Beispiel PAVR *percutaneous aortic valve replacement*)*.* Die Verankerung in der Gefäßwand erfolgt über eine Stützstruktur für die eigentliche Herzklappe, zum Beispiel über ein metallisches Geflecht, dessen Aufbau und Materialwahl dem eines Stents angelehnt ist. Die Stützstruktur kann selbstexpandierend sein oder wird mittels eines Ballonkatheters aufgeweitet.

Herkömmliche Transkatheter-Herzklappenprothesen besitzen demnach eine Stützstruktur, die aus einer für ein minimal-invasives Einführen ausgelegten ersten Größe in eine funktionale zweite Größe aufweitbar ist. An dieser Stützstruktur ist die eigentliche Herzklappe fixiert, die zunächst eine zum minimal-invasiven Einführen ausgelegte erste Form einnimmt, die im Zuge der Implantation zur funktionalen zweiten Form aufweitbar ist. Beispielsweise besteht die Herzklappe aus mehreren flexiblen Blättern, die sich je nach den wirkenden Blutflusskräften öffnen oder schließen. Eine derartige Transkatheter-Herzklappenprothese mit einer biologischen Herzklappe ist beispielsweise EP 1 267 753 B1 zu entnehmen.

Der nächste Stand der Technik ist Dokument WO2009/038761 A1, welches ein Klappensegel offenbart, das aus einer elastischen Matrix besteht, in welche ein Gewebe eingebettet ist. Es offenbart keine zusätzliche lokale Verstärkung des Gewebes durch Fasern.
Eine lokale Verstärkung von Klappensegeln durch das Einbetten von Fasern in eine Matrix ist aus den Dokumenten US2003/0078652, US2006/0190074 und WO02/24119 bekannt.

In der Praxis ist es notwendig, Transkatheter-Herzklappenprothesen in verschiedensten Größen bereitzustellen, denn die benötigten Klappendurchmesser differieren erheblich unter den Empfängern. Die Dimension der Klappenstruktur für eine flexible Herzklappe muss demnach sehr genau auf den Montagedurchmesser angepasst sein, um der Bildung von Falten einerseits und einer Leckage andererseits vorzubeugen. Hierbei ist zu beachten, dass jede Verformungsarbeit, die geleistet werden muss, um die Klappe zu schließen, zu einem Verlust an Pumpvolumen führt. Bei der Anpassung bekannter Herzklappen an die am Implantationsort erforderlichen Dimensionen muss dieser Nachteil bisher in Kauf genommen werden.

Aufgabe der vorliegenden Erfindung ist es, einen oder mehrere Nachteile des Standes der Technik zu vermindern oder zu vermeiden. Insbesondere ist es eine Aufgabe der vorliegenden Erfindung eine mechanische Transkatheter-Herzklappenprothese bereitzustellen, die aus einem künstlichen Material besteht und die ein für den Bestimmungszweck optimiertes mechanisches Verhalten zeigt.

Die vorliegende Erfindung ist definiert in Anspruch 1 und löst diese Aufgabe durch Bereitstellung einer mechanischen Transkatheter-Herzklappenprothese, die eine Stützstruktur, die aus einer für ein minimal-invasives Einführen ausgelegten ersten Größe in eine funktionale zweite Größe aufweitbar ist; und eine flexible, aus mehreren Blättern bestehende Herzklappe aus einem Compositmaterial, das ein in eine elastische Matrix gebettetes Gewebe beinhaltet, welches lokal durch Fasern verstärkt ist, umfasst.

Der Erfindung liegt der Gedanke zugrunde, eine Herzklappe so zu konstruieren, dass viskoelastische Effekte nutzbar sind, um eine automatische Anpassung an die Gefäßdimensionen zu realisieren. Der Effekt beruht auf einem Spannungsabbau in den Bereichen, die durch Schließkräfte der Herzklappe maßgeblich geprägt werden, bzw. diese aufnehmen. Allerdings muss auch die Grundstruktur der Herzklappe im druckbelasteten Zustand erhalten bleiben - dieses Ziel wird vorliegend durch eine lokale Faserverstärkung in speziellen (diesen) Bereichen erreicht. Erfindungsgemäß ist dazu vorgesehen, dass die an einer herkömmlichen Stützstruktur befestigte flexible Herklappe aus einem künstlichen Material besteht, das auf Basis eines Gewebes hergestellt wird. Dieses Gewebe wird zur Herstellung einer geschlossenen, dichten Membran in ein viskoelastisches Material eingebettet. Durch lokale Faserverstärkung der Herzklappe in Bereichen, in welchen hohe Belastungen in funktionsgemäßen Verbrauch zu erwarten sind, kann diese ausgesteift werden oder auch eine viskoelastische Verformung unterdrückt werden. Das viskoelastische Matrixmaterial kann in nicht effektiv faserverstärkten Bereichen durch viskoelastische Dehnung, einem Retardationsphenomen, im druckbelasteten Zustand geformt werden. Durch textile Prinzipien können Substrate erzeugt werden, welche vorteilhafte anisotrope Eigenschaften aufweisen. So können z.B. Gewebe mit Faseranordnungen als Substrat für die Herstellung genutzt werden, welche in eine Richtung dehnbar und somit "anpassbar" sind, in eine andere Richtung allerdings nicht dehnbar und somit druckfest und dimensionsstabil bleiben.

Bei Polymeren, wie auch metallischen Konstrukten wird davon ausgegangen, dass die Hysterese zwischen belastetem Zustand und entlastetem Zustand eine Energieaufnahme des Materials bedeutet, welche die zeitliche Belastbarkeit des Systems begrenzt. Andererseits bietet die Viskoelastizität von polymeren Werkstoffen auch die Möglichkeit ein technisches Konstrukt an Dimensionen anzupassen. Im Falle der Herzklappe, die im geschlossenen Zustand unter starker mechanischer Spannung steht, in diesem Zustand somit auch viskoelastisch geprägt werden kann, ist diese technische Materialeigenschaft gezielt nutzbar um eine Dimensionsanpassung zu realisieren. Ein isotropes viskoelastisches Klappenmaterial würde allerdings langfristig dem Belastungsdruck der Herzklappe nachgeben. Daher wird ein anisotropes Konstrukt erforderlich sein, welches die Freiheit besitzt sich anzupassen, dessen Anisotropie aber so beschaffen sein sollte, dass sie in Verformungsrichtungen die für Sperrfunktion der Herzklappe essentiell sind, keine oder nur minimale Verformungen ermöglicht. Mit anderen Worten, idealer Weise sollte Bereiche der künstlichen Herzklappe, die bei der Dimensionierung verformt werden, eine möglichst hohe Anpassungsfähigkeit aufweisen. Dagegen sollten die Bereiche der Herzklappe, die nach erfolgter Dimensionsanpassung in bestimmungsgemäßer Funktion besonders belastet werden, in Ihren Dimensionen beibehalten werden, um eine langfristige Funktion des künstlichen Gebildes zu gewährleisten. Es kann ferner der Bedarf bestehen, sowohl die Steifigkeit, wie auch die Festigkeit der künstlichen Herzklappen lokal zu beeinflussen. Um die minimal-invasive Anwendung zu gewährleisten, sollten stets Werkstoffe maximaler Festigkeit mit Werkstoffen sehr hoher Festigkeit und Dehnung kombiniert werden, damit die mechanische Funktion bei einem Minimum an Schichtdicke realisierbar ist.

Nach einer bevorzugten Ausführungsform der Erfindung ist die Herzklappe in einem an die Stützstruktur grenzenden Randabschnitt durch die Fasern verstärkt. Ferner ist bevorzugt, wenn die Herzklappe eine triskupide Kontur aufweist.

Weitere Aspekte, die unter Bezugnahme auf die erfindungsgemäße Lehre von Bedeutung sind:
a) Die typische Eigenschaft einer Stützstruktur auf Basis einer Nitinolkonstruktion, welche erhebliche Kräfte auf die Gefäßwand ausübt, ist eine Migration der Struktur in tiefere Regionen des Gewebes. Die Dimension und Positionierung der Stützstruktur im Gefäß kann dadurch kritisch werden, da sich Abweichungen in dem Durchmesser direkt auf die Schließfähigkeit des Klappengebildes auswirken. Die Herzklappen befinden sich in der Ebene eines Bindegewebes, dem sogenannten Herzskelett, welches die Migration gut begrenzen kann. Wird diese Ebene verlassen, ist die Toleranz bezüglich Migration deutlich geringer.
b) Eine Nachbildung der Herzklappenstruktur aus einem homogenen Material bedingt die Anwendung eines elastischen Polymeren. Elastomere weisen jedoch immer ein viskoelastisches Verhalten auf, welches deren Formbeständigkeit limitiert. Auch in vielen technischen Anwendungen, die Standfestigkeit erfordern, werden in der Regel Elastomere mit möglichst ideal elastischem Verhalten (d. h. geringe Viskoelastizität) in Faserverbünden oder gar Stahlverbünden realisiert, um langfristig die Formbeständigkeit zu garantieren. Während es erwünscht sein könnte, dass sich die einzelnen flexiblen Blätter einer Herzklappen zueinander orientieren und anpassen, ist die Formbeständigkeit des Membransegmentes, welches maßgeblich die Kräfte im geschlossenen Zustand der Herzklappe aufnimmt, ebenfalls gefordert, um die funktionelle Form über einen längeren Zeitraum zu gewähren.
c) Um sowohl ein Schließen, wie auch ein Öffnen der Klappe bei sehr geringem Differenzdruck zu ermöglichen, muss die Klappe in der Lage sein, lokal sehr hohe Biegungen zu realisieren. Es kann notwendig sein, diese Bereiche gezielt mit einer Verstärkung zu versehen, die sowohl eine hohe Festigkeit, wie auch ideal elastisches Verhalten aufweisen sollte. Da die Bedingung besteht, dies räumlich auch mit sehr geringen Materialdicken zu realisieren, ist technisch der Weg einer Faserverstärkung vorteilhaft.
d) Um Bewegungspunkte und Knickpunkte für ein vorteilhaftes Klappenverhalten zu definieren, können Bereiche der Herzklappe gezielt auf textilem Wege durch Fasern verdickt und ausgesteift werden.

Unter dem erfindungsgemäß im Compositmaterial eingesetzten Gewebe wird vorliegend ein textiles Flächengebilde aus mindestens zwei unterschiedlichen Faserorientierungen verstanden. Dies kann durch eine Gewebestruktur oder auch einzelne Faserlagen erfolgen, da davon ausgegangen wird, dass der endgültige Verbund durch eine polymere Matrix gewährleistet ist. Unter den Begriff Gewebe fallen insbesondere verkreuzte Fadensysteme, aber auch ein Produkt aus dem Ineinanderschlingen mehrerer Stränge aus biegsamem Material (Geflecht).

Das Gewebe wird im Produkt über eine polymere Matrix fixiert. Die Matrix besteht vorzugsweise aus einem Polyurethan, da Polyurethane in der Regel einen sehr guten Kompromiss aus Festigkeit und Elastizität gewähren. Dieser Eigenschaften sind insbesondere notwendig, um geringe Dimensionen, wie z.B. geringe Schichtdicken, zu realisieren.

Die erfindungsgemäße Faserverstärkung kann unter Rückgriff auf bekannte Technologien erfolgen. So sind beispielsweise textile Faserlegetechnologien und Aufstickverfahren bekannt. Um einen möglichst dünnen, belastbaren textilen Grundkörper zu erhalten, können hochfeste, wie auch dauerbelastungsbeständige Fasern verwendet werden. Die Fasern bestehen vorzugsweise aus hochfestem Polyethylen (PE). Beispielsweise können PE-Fasern, die unter dem Namen Dyneema, welche auch für Implantatanwendungen vertrieben werden (erhältlich bei der Firma DSM N.V.), Einsatz finden.

Das Verbundmaterial kann ferner eine Beschichtung zur Verbesserung der Biokompatibilität aufweisen. Die Beschichtung enthält oder besteht aus einem biokompatiblen Werkstoff, der beispielsweise die Zellbesiedlung verbessert. Ein biokompatibler Werkstoff ist ein nicht lebendes Material, das für eine Anwendung in der Medizin eingesetzt wird und mit biologischen Systemen in Wechselwirkung tritt. Grundvoraussetzungen für den Einsatz eines Werkstoffes, der bei bestimmungsgemäßer Verwendung mit der Körperumgebung in Kontakt steht, ist dessen Biokompatibilität (Körperverträglichkeit). Unter Biokompatibilität wird die Fähigkeit eines Werkstoffes verstanden, in einer spezifischen Anwendung eine angemessene Gewebereaktion hervorzurufen. Dies beinhaltet eine Anpassung der chemischen, physikalischen, biologischen und morphologischen Oberflächeneigenschaften eines Implantates an das Empfängergewebe mit dem Ziel einer klinisch erwünschten Wechselwirkung. Bevorzugt werden biokompatible Werkstoffe für die Beschichtung verwendet, die im Wesentlichen bioinert sind. Unter bioinerten Werkstoffen werden solche Werkstoffe verstanden, die nach der Implantation über die geplante Standzeit der Herzklappenprothese im Wesentlichen intakt bleiben und keine signifikante Biokorrosion zeigen. Als Prüfmedium zur Testung des Korrosionsverhaltens eines in Frage kommenden Werkstoffs dient künstliches Plasma, wie es nach EN ISO 10993-15:2000 für Biokorrosionsuntersuchungen vorgeschrieben ist (Zusammensetzung NaCl 6,8 g/l, CaCl₂ 0,2 g/l, KCl 0,4 g/l, MgSO₄ 0,1 g/l, NaHCO₃ 2,2 g/l, Na₂HPO₄ 0,126 g/l, NaH₂PO₄ 0,026 g/l). Eine Probe des zu untersuchenden Werkstoffs wird dazu in einem verschlossenen Probenbehälter mit einer definierten Menge des Prüfmediums bei 37 °C gelagert. In zeitlichen Abständen - abgestimmt auf das zu erwartende Korrosionsverhalten - von wenigen Tagen bis zu mehreren Monaten oder Jahren werden die Proben entnommen und in bekannter Weise auf Korrosionsspuren untersucht. Das künstliche Plasma nach EN ISO 10993-15:2000 entspricht einem blutähnlichen Medium und stellt damit eine Möglichkeit dar, eine physiologische Umgebung im Sinne der Erfindung reproduzierbar nachzustellen. Ein Werkstoff gilt insbesondere dann als bioinert, wenn der Werkstoff in oben genanntem Test nach Ablauf von 12 Monaten zu weniger als 10% korrodiert ist.

Die Erfindung wird nachfolgend anhand eines Ausführungsbeispiels und den dazugehörigen Figuren näher erläutert. Es zeigen:
- Fig. 1: zeigt das Compositmaterial in einem Ausführungsbeispiel,
- Fig. 2: illustriert den Zuschnitt des Compositmaterials aus Figur 1,
- Fig. 3: skizziert die dreidimensionale Verformung des Materials zur Herstellung der Herzklappe,
- Fig. 4: visualisiert die geometrischen Begrenzungen an der flachen Struktur.

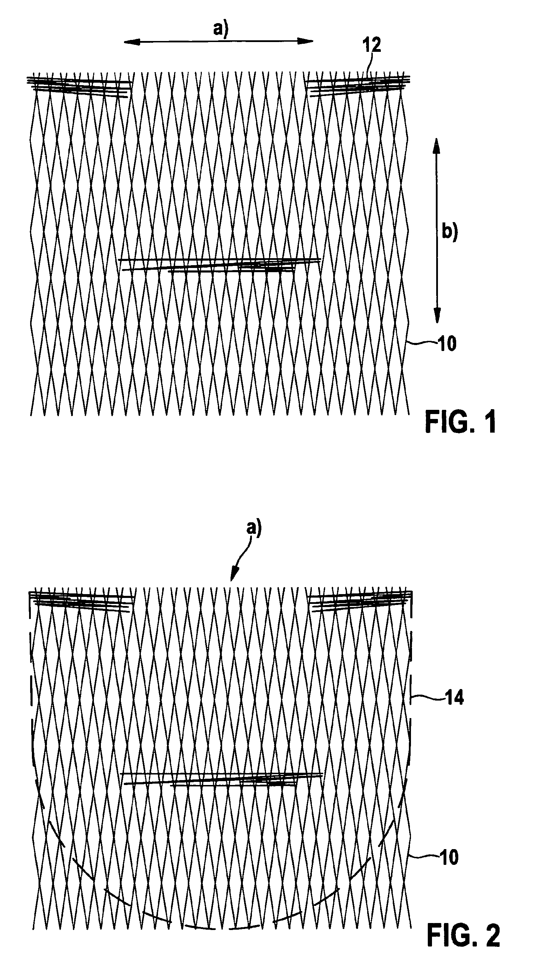

Figur 1 illustriert ein Compositmaterial, das anisotrope mechanische Eigenschaften besitzt. Das Compositmaterial umfasst ein Gewebe 10, das durch eine elastomere Matrix fixiert ist. In Richtung a) ist das Gewebe 10 elastischer als in Richtung b). Soll aus einer Oberkante dieses Gebildes auch die Oberkante einer flexiblen, aus mehreren Blättern bestehenden Herzklappe gebildet werden, würde sich ein großer Teil der Biegung der Klappe, wie auch der Kraftübertragung zu dem Klappenboden, in den Seitenbereichen konzentrieren. In analoger Weise kann anstelle eines Gewebes auch ein Fasergelege verwendet werde.

In das Gewebe 10 sind deshalb Fasern 12 mit skizzierter Orientierung eingezogenen. Zwischen den oberen Randbereichen, welche faserverstärkt sind, bleibt dann noch ein mittlerer Bereich, welcher in Richtung a) dehnbar verbleibt - in Richtung b) allerdings in seiner Dehnung stark durch die Fasern 12 begrenzt ist.

In dem mittleren bis unteren Bereich des Gewebes 10 soll sich die zylinderförmige Form der Herzklappe unter Druck ausbilden. Ausgehend davon, dass die Herzklappe in den Randbereichen zu fixieren ist und das Gewebe 10 aus dem oberen Bereich Kräfte in die Mitte übertragen kann, ist es sinnvoll den mittleren Bereich ebenfalls zum Teil durch eine Faserverstärkung zu verfestigen.

Hierdurch erhält man einen unteren Bereich der Herzklappe, der in seiner Struktur komplett von Fasern 12 durchzogen und in Verformung begrenzt ist. Dieser untere Bereich soll die Drucklast der geschlossenen Herzklappe tragen, ohne dass eine viskoelastische Verformung möglich ist. Es lässt sich damit ein Compositmaterial bereitstellen, welches viskoelastisch verformbare, wie auch über längere Zeit belastungsstabile Bereiche umfasst.

Um die Herzklappe zu formen, muss das Compositmaterial entlang der Schnittlinie 14 aus Figur 2 zurechtgeschnitten werden.

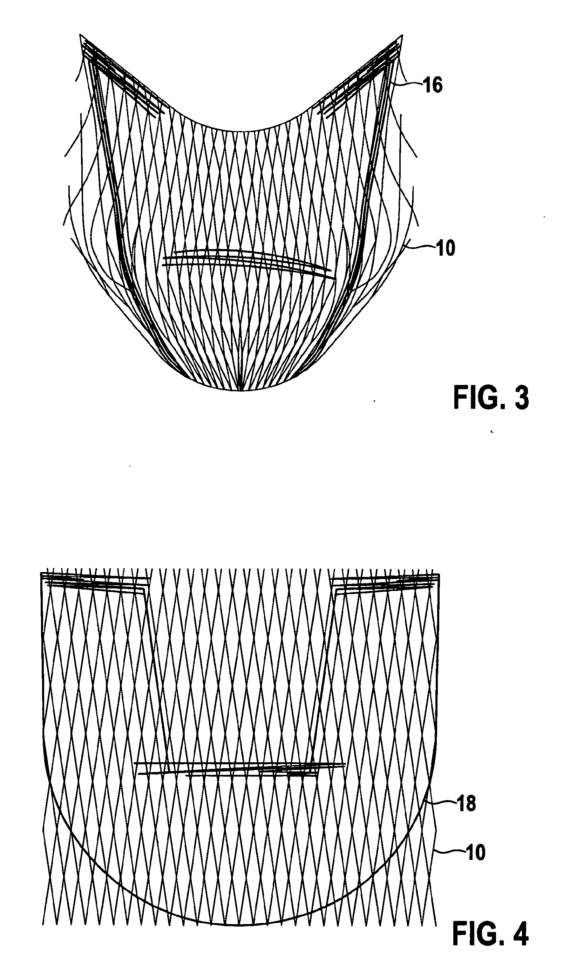

Figur 3 illustriert eine Verformung des Compositmaterials nach unten und eine Vorformung auf den 120° Winkel des oberen Herzklappenrandes. Die Linie 16 soll den Bereich darstellen, an welchem die Herzklappe später an eine zylindrische Stützstruktur angebunden wird.

In Figur 4 werden durch eine Linie 18 die geometrischen Begrenzungen an der flachen Struktur visualisiert.

Durch die aufgezeigte Faserverstärkung soll die mechanische Langzeitstabilität der Herzklappe verbessert werden. Die lokale Verstärkung wird aber auch eine Aussteifung zur Folge haben, welche genutzt werden kann, um die dynamische Bewegungsmechanik zu verbessern. Die Biegebereiche der einzelnen Blätter der Herzklappe werden auf die Randbereiche der Herzklappe derart konzentriert, dass im Falle der Öffnung der Herzklappe ein möglichst großer Durchmesser freigegeben wird.

## Patentansprüche

1. Mechanische Transkatheter-Herzklappenprothese umfassend:
eine Stützstruktur, die aus einer für ein minimal-invasives Einführen ausgelegten ersten Größe in eine funktionale zweite Größe aufweitbar ist; und
eine flexible, aus mehreren Blättern bestehende Herzklappe aus einem Compositmaterial, das ein in eine elastische Matrix gebettetes Gewebe (10) beinhaltet, **dadurch gekennzeichnet, dass** das Gewebe lokal durch Fasern (12) verstärkt ist.

2. Herzklappenprothese nach Anspruch 1, bei dem die Herzklappe in einem an die Stützstruktur grenzenden Randabschnitt durch die Fasern (12) verstärkt ist.

3. Herzklappenprothese nach Anspruch 1, bei dem die Herzklappe eine triskupide Kontur aufwei st.

4. Herzklappenprothese nach Anspruch 1, bei dem die Matrix aus einem Polyurethan besteht.

5. Herzklappenprothese nach Anspruch 1, bei dem die Fasern (12) aus hochfestem Polyethylen (PE) bestehen.

## Claims

1. A mechanical transcatheter heart valve prosthesis comprising:
a support structure, which can be expanded from a first size designed for minimally invasive insertion, into a functional second size; and
a flexible heart valve, which comprises multiple leaflets and is made of a composite material containing a woven fabric (10) that is embedded in an elastic matrix, **characterized in that** the woven fabric is locally reinforced by fibers (12).

2. The heart valve prosthesis according to claim 1, wherein the heart valve is reinforced by the fibers (12) in an edge section located adjacent to the support structure.

3. The heart valve prosthesis according to claim 1, wherein the heart valve has a tricuspid contour.

4. The heart valve prosthesis according to claim 1, wherein the matrix is made of a polyurethane.

5. The heart valve prosthesis according to claim 1, wherein the fibers (12) are made of high-density polyethylene (PE).

## Revendications

1. Prothèse mécanique de valvule cardiaque transcathéter, comprenant :
une structure d'appui apte à être élargie, d'une première taille conçue pour une introduction peu invasive à une deuxième taille fonctionnelle ; et
une valvule cardiaque souple, constituée de plusieurs feuilles d'un matériau composite contenant un tissu (10) enrobé dans une matrice élastique, **caractérisée en ce que** le tissu est localement renforcé par des fibres (12).

2. Prothèse de valvule cardiaque selon la revendication 1, dans laquelle la valvule cardiaque est renforcée par les fibres (12) dans une section de bord adjacente à la structure d'appui.

3. Prothèse de valvule cardiaque selon la revendication 1, dans laquelle la valvule cardiaque présente un contour triscupide.

4. Prothèse de valvule cardiaque selon la revendication 1, dans laquelle la matrice est constituée de polyuréthane.

5. Prothèse de valvule cardiaque selon la revendication 1, dans laquelle les fibres (12) sont constituées de polyéthylène (PE) hautement fort.
